# EUROPÄISCHE PATENTSCHRIFT

(11) **EP 1 013 774 B1**
(45) Veröffentlichungstag und Bekanntmachung des Hinweises auf die Patenterteilung: **11.10.2006**
(21) Anmeldenummer: 99124783.4
(22) Anmeldetag: 14.12.1999
(51) Int. Cl.: C12Q 1/50, C12Q 1/48, C12Q 1/32

(54) **Flüssigreagenz für den Nachweis von Kreatinkinase**
Liquid reagent for the detection of creatine kinase
Réactif liquide pour la détection de créatine kinase

(30) Priorität: 22.12.1998 DE 19859261
(43) Veröffentlichungstag der Anmeldung: 28.06.2000
(73) Patentinhaber: Olympus Diagnostica GmbH, 20097 Hamburg (DE)
(72) Erfinder: Gunzer, Gerhard, Dr., Ennis, Co Clare (IE); Kretzschmar, Frank, Dr., 01809 Dohna OT, Borthen (DE); Wrynn, Katy, Ennis, Co. Clare (IE)
(74) Vertreter: Emmel, Thomas

(56) Entgegenhaltungen:
- EP-A- 0 721 986
- US-A- 4 547 461
- PATENT ABSTRACTS OF JAPAN vol. 1999, no. 03, 31. März 1999 (1999-03-31) & JP 10 327895 A (NIPPON SHOJI KK), 15. Dezember 1998 (1998-12-15)

## Beschreibung

Die Erfindung bezieht sich auf ein Flüssigreagenz nach dem Oberbegriff des Anspruches 1.

Kreatinkinase katalysiert im Körper die ATP-abhängige Phosphorylierung von Kreatin zu Kreatinphosphat (Hin-und Rückreaktion). Im Körper liegt Kreatinkinase u.a. im Gehirn, im Herzmuskel und der Skelettmuskulatur vor. Erhöhte Kreatinkinase-Werte im Herzmuskel finden sich bei Herzinfarkt, in der Skelettmuskulatur deuten sie auf Muskelerkrankungen (z.B. Muskeldystrophie) hin. Niereninsuffizienz z.B. geht mit einem Anstieg der Kreatinkinase-Aktivität im Serum einher etc.

Die Kreatinkinase-Aktivität ist damit ein wichtiger klinischer Untersuchungsparameter und wird routinemäßig bestimmt.

Ein gängiges Nachweissystem für die Kreatinkinase-Aktivität arbeitet nach folgendem Reaktionsschema:

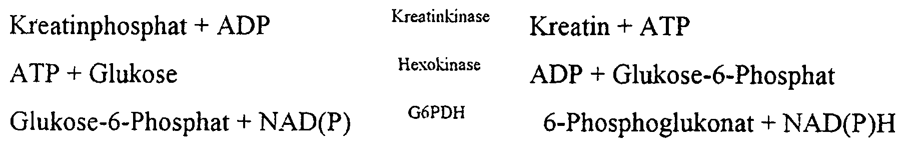

Bei diesem Nachweis wird die Kreatinkinase-Aktivität indirekt über den NAD(P)H-Gehalt durch Absorptionsmessung bei 340 nm bestimmt.

Herkömmliche Flüssigreagenzien, mit denen ein Nachweis von Kreatinkinase z.B. nach dem obigen Reaktionsschema möglich ist, enthalten die erforderlichen Substrate, Kosubstrate und Enzyme und ggf noch weitere z.B stabilisierende oder bakterizide Substanzen etc. In vielen Fällen werden die für die Nachweisreaktionen erforderlichen Reagenzien auf 2 separate Reagenzansätze dergestalt verteilt, daß während der Lagerung keine ungewollten enzymatischen Abbau-Reaktionen ablaufen. Die Reagenzansätze werden für den Nachweis miteinander zu dem Flüssigreagenz (Testreagenz) vermischt, und erst dann starten die Nachweisreaktionen in Abhängigkeit von der Kreatinkinase-Aktivität in der untersuchten Probe.

Das beschriebene Nachweisverfahren ist relativ zuverlässig und Grundlage vieler Kreatinkinaseaktivitätstests.

Ein Problem ist allerdings, daß Kreatinkinase z.B im Serum relativ rasch inaktiviert wird. Aus diesem Grund enthalten gattungsgemäße Flüssigreagenzien einen Reaktivator für Kreatinkinase. Als Reaktivator werden SH-Gruppen enthaltende Komponenten, standardmäßig N-Acetylcystein (im folgenden als NAC abgekürzt) eingesetzt, da es sich für die Gefriertrocknung eignet.

In der Literatur wird NAC allerdings nur als bedingt geeigneter Reaktivator gewertet (Morin, CLINICAL CHEMISTRY, Vol. 23, Nr. 9, September 1977, Seiten 1569 - 1575). Es hat sich insbesondere gezeigt, daß die Wirkung von NAC als Reaktivator nach längerer Lagerung durch Bildung von Inhibitoren abnimmt, weswegen in manchen bekannten NAC enthaltenden Nachweissystemen spezielle Substanzen zur Stabilisierung des NAC vorgesehen werden.

In der zitierten Veröffentlichung werden daher andere Reaktivatoren, insbesondere Thioglycerol, empfohlen, dem eine gegenüber NAC deutlich erhöhte Reaktivierungsleistung und Lagerstabilität zugemessen wird.

Es hat sich allerdings bei Untersuchungen der Anmelderin gezeigt, daß auch der Einsatz von Thioglycerol in Flüssigreagenzien zum Nachweis von Kreatinkinase, insbesondere im Hinblick auf die Stabilität, nicht unproblematisch ist.

Thioglycerol wird daher in bekannten Flüssigreagenzien vorwiegend separat eingesetzt. So offenbart z.B. die DE 31 38 602 ein Flüssigreagenz mit zwei Reagenzansätzen, dessen einer Reagenzansatz im wesentlichen ein Thioglycerol-Puffer ist. In diesem Ansatz ist Thioglycerol nicht als Reaktivator vorgesehen. Vielmehr wird der Thioglycerol enthaltene Reagenzansatz einer Probe vor der eigentlichen Analyse zur Trübungsbeseitigung zugesetzt. Die geklärte Probe wird dann mit dem zweiten, die eigentlichen Nachweisreagenzien enthaltenen Reagenzansatz vermischt.

Die WO 95/30769 beschreibt ein Flüssigreagenz, dessen einer Ansatz als Aktivatorlösung dient und der als Hauptbestandteil Thioglycerol enthält. Die weiteren für den Nachweis erforderlichen Substanzen sind in den anderen Reagenzansätzen enthalten. Bei dem beschriebenen Flüssigreagenz erreicht man eine verbesserte Stabilität des Thioglycerols. Allerdings wird durch den zusätzlichen Ansatz (in aller Regel besteht das bekannte Flüssigreagenz aus insgesamt drei separaten Ansätzen) das Handling schwieriger.

Aufgabe der Erfindung ist es daher, ein Flüssigreagenz der eingangs genannten Art zu schaffen, das Thioglycerol als Reaktivator enthält, langzeitstabil ist und das einen Nachweis mit geringem Aufwand ermöglicht.

Gelöst wird die Aufgabe mit einem Reagenz, das die kennzeichnenden Merkmale des Anspruches 1 aufweist.

Danach ist ein Flüssigkeitsreagenz zum Nachweis von Kreatinkinase-Aktivität vorgesehen, das folgende Substanzen enthält: Glukose-6-Phosphatdehydrogenase (im folgenden mit G6PDH abgekürzt), Hexokinase, Kreatinphosphat, Glukose, ADP, NAD(P) und Thioglycerol, wobei die Substanzen erfindungsgemäß dergestalt auf zwei separate Reagenzansätze aufgeteilt sind, daß Thioglycerol und mindestens eine weitere Substanz in dem einen Ansatz und G6PDH und eventuell verbleibende Substanzen in dem anderen Reagenzansatz enthalten sind.

Es hat sich gezeigt, daß durch die erfindungsgemäße Trennung von G6PDH von dem Thioglycerol und gegebenenfalls weiteren üblicherweise eingesetzten Substanzen wie NADP, Glukose, Hexokinase, ADP, AMP oder MG die Lagerstabilität der beiden Reagenzansätze und damit des Flüssigreagenzes insgesamt bedeutend gesteigert werden kann.

Weitere Ausgestaltungen der Erfindung sind in den Unteransprüchen angegeben.

Im Hinblick darauf, daß die Stabilität zumindest einiger der im Flüssigreagenz enthaltenen Substanzen pH-Wert abhängig ist, sieht eine weitere Ausgestaltung der Erfindung vor, daß die Reagenzansätze unterschiedliche pH-Werte aufweisen. Bei entsprechender Verteilung der Substanzen auf die Ansätze kann auf diese Weise die Lagerstabilität optimiert werden.

Einer der Reagenzansätze wird dabei bevorzugt auf einen schwach basischen pH-Wert in einem Bereich zwischen 8,0 und 10,0 (gemessen bei 20° C) eingestellt, während der andere Reagenzansatz einen pH-Wert zwischen 5,5 und 7,0 aufweist. Darauf basierend sieht eine weitere Ausgestaltung der Erfindung vor, daß G6PDH und/oder ADP vorteilhafterweise in den schwach basischen Reagenzansatz gegeben werden, da diese Substanzen bei höheren pH-Werten stabiler sind.

Vorzugsweise werden die pH-Werte und Mengenverhältnisse der Reagenzansätze so gewählt, daß nach ihrer Vermischung sich ein pH-Wert von ca. 6,0 - 7,0, insbesondere 6,5, in dem fertigen Flüssigreagenz (Testreagenz) ergibt.

Besonders vorteilhaft wird in diesem Zusammenhang in dem einen Reagenzansatz ein zwitterionischer Puffer vorgesehen, dessen einer pK-Wert bei pH-Werten zwischen 8,0 und 10,0 und dessen anderer pK-Wert zwischen ca. 5,5 und 7,0 liegt. Während der Lagerung stellt dieser Puffer den Reagenzansatz auf einen basischen pH-Wert ein und stellt sich nach Vermischung mit dem anderen Reagenzansatz auf den gewünschten Nachweis-pH-Wert von 6,0 - 7,0 um, insbesondere auf 6,5. Bei Verwendung eines zwitterionischen Puffers, z.B. eines Bis-Tris-Propan-Puffers mit pK-Werten, die im Bereich der oben angegebenen pH-Werte liegen, läßt sich das Puffervolumen gegenüber der Verwendung herkömmlicher Puffer verringern.

Weiterhin wurde festgestellt, daß die herkömmlicherweise eingesetzte G6PDH in Gegenwart von Glukose und NAD(P) ungewollte Reaktionen zeigt. Deswegen sieht eine weitere vorteilhafte Ausgestaltung der Erfindung vor, daß die drei genannten Substanzen nicht zusammen in einem Reagenzansatz vorgesehen sind.

Ein weiteres Problem betrifft die Stabilität des Thioglycerols. Die herkömmlicherweise zur Stabilisierung von Enzymen und von SH-Verbindungen eingesetzten Proteine wie z.B. BSA oder Gelatin führen bei Thioglycerol eher zu einer Abnahme der Stabilität. Anstelle von Proteinen werden daher vorzugsweise Zukkerderivate, wie z.B. Mannitol oder Polyethylenglykol 6000 oder auch langkettige Alkylverbindungen, erhältlich unter dem Markennamen Synperonic, zugesetzt, die die Stabilität der Hilfsenzyme (Hexokinase und G6PDH) erhalten und keine Thioglycerol abbauenden Bestandteile enthalten.

Weiterhin hat sich gezeigt, daß Hexokinase durch Gegenwart von Glukose stabilisiert wird. Deswegen werden diese beiden Substanzen bevorzugt in einem Reagenzansatz vorgesehen.

Das erfindungsgemäße Reagenz kann weitere Substanzen enthalten wie z.B. EDTA als Oxidationsschutz, NaN₃ und Gentamycin Sulfat als bakterizide bzw. fungizide Zusätze. Weiterhin können AMP und AP₅A zugesetzt werden, die als kompetitive Inhibitoren für die Adenylatkinase dazu beitragen, das Risiko falsch positiver Signale zu verringern. Weiterhin kann Magnesiumacetat enthalten sein, das mit ADP einen Vorkomplex ausbildet und dadurch die Aktivität der Kreatinkinase steigert.

In dem Ansatz, der kein Thioglycerol enthält, kann schließlich noch ein Protein z.B. Gelatin oder BSA zur Stabilisierung von G6PDH vorgesehen werden.

Schließlich ist die Erfindung nicht auf die ausschließliche Verwendung von Thioglycerol beschränkt. Es ist auch möglich, zusätzlich weitere Reaktivatoren, z.B. das eingangs angesprochene NAC vorzusehen.

Erfindungsgemäß einsetzbare Nachweisreagenzien können z.B. folgende Zusammensetzungen aufweisen:

| **Reagenzansatz 1** | **Bevorzugte Konz. im Ansatz** | | **Möglicher Konz. Bereich** | |
|---|---|---|---|---|
| Imidazol-Puffer | 82 | mmol/L | 50-150 | mmol/L |
| EDTA | 2,5 | mmol/L | 2-3 | mmol/L |
| Glukose | 25 | mmol/L | 20-50 | mmol/L |
| Magnesium Azetat | 12.5 | mmol/L | 10-15 | mmol/L |
| AMP | 6.5 | mmol/L | 5-10 | mmol/L |
| AP₅A | 0,013 | mmol/L | 0,01-0,05 | mmol/L |
| NADP | 2.5 | mmol/L | 2-5 | mmol/L |
| NaN₃ | 0.095 | % | | |
| Gentamycin Sulfat | 0.005 | % | | |
| PEG 6000 | 2.0 | % | 0,5-5 | % |
| Mannitol | 1,0 | % | 1-5 | % |
| Synperonic | 0,1 | % | 0,05-0,2 | % |
| Hexokinase | 7,5 | kU/L | 3-10 | kU/L |
| Thioglycerol | 26 | mmol/L | 20-100 | mmol/L |
| NAC | 0.2 | mmol/L | 0-2,0 | mmol/L |

| | | | | |
|---|---|---|---|---|
| Der pH-Wert wird auf 6.20 ± 0.20 bei 20° C eingestellt. | | | | |

| **Reagenzansatz 2:** | | | | |
|---|---|---|---|---|
| G6P-DH | 20 | kU/L | 10-30 | kU/L |
| Bis-Tris Propan | 70 | mmol/L | 50-100 | mmol/L |
| CP | 50 | mmol/L | 30-70 | mmol/L |
| ADP | 5 | mmol/L | 3-6 | mmol/L |
| NaN₃ | 0,095 | % | | |
| Gentamycin Sulfat | 0,005 | % | | |
| Gelatine | 0,2 | % | 0,1-0,5 | % |
| Mannitol | 1 | % | 1-5 | % |
| Synperonic | 0.1 | % | 0,05-0,2 | % |

Der pH-Wert wird auf 9,20 ± 0,20 bei 20° C eingestellt.

Die Reagenzansätze werden im Verhältnis 4+1 (4 Teile Reagenzansatz 1 + 1 Teil Reagenzansatz 2) miteinander zu dem Testreagenz vermischt.

Die obigen Angaben betreffen eine bevorzugte Ausführung für das erfindungsgemäße Reagenz. Es versteht sich, daß die Erfindung auch andere Zusammensetzungen abdeckt, die weniger oder auch andere Substanzen enthalten, z.B. andere Puffer oder Stabilisierungsreagenzien etc.

## Patentansprüche

1. Flüssigreagenz zum Nachweis von Kreatinkinase-Aktivität, in dem zumindest folgende Substanzen enthalten sind: G6PDH, Hexokinase, Kreatinphosphat, Glukose, ADP, NAD(P) und Thioglycerol, wobei die Substanzen auf zwei separate Reagenzansätze aufgeteilt sind und die Reagenzansätze für den Nachweis miteinander vermischt werden, **dadurch gekennzeichnet, daß** G6PDH in dem einem Ansatz und Thioglycerol mit mindestens einer weiteren Substanz in dem anderen Ansatz enthalten ist:

2. Reagenz nach Anspruch 1, **dadurch gekennzeichnet, daß** einer der Reagenzansätze auf einen schwach sauren pH-Wert zwischen 5,5 und 7,0 und der andere auf einen schwach basischen pH-Wert zwischen 8,0 und 10,0 eingestellt ist.

3. Reagenz nach Anspruch 2, **dadurch gekennzeichnet, daß** die Mengenverhältnisse und pH-Werte der Reagenzansätze so gewählt sind, daß sich nach ihrer Vermischung im Testreagenz ein pH-Wert in einem Bereich von 6,0 - 7,0, insbesondere von ca. 6,5 ergibt.

4. Reagenz nach einem der Ansprüche 2 oder 3, **dadurch gekennzeichnet, daß** in dem basischen Reagenzansatz ein zwitterionischer Puffer vorgesehen ist, dessen einer pK-Wert bei Werten zwischen pH 8,0 und 10,0 und dessen anderer pK-Wert bei pH-Werten zwischen 5,5 und 7,0 liegt.

5. Reagenz nach einem der Ansprüche 2 bis 4, **dadurch gekennzeichnet, daß** G6PDH und ADP in dem Ansatz mit dem basischen pH-Wert vorgesehen sind.

6. Reagenz nach einem der Ansprüche 1 bis 5, **dadurch gekennzeichnet, daß** NAD(P), Glukose und G6PDH nicht gemeinsam in einem Ansatz vorgesehen sind.

7. Reagenz nach einem der Ansprüche 1 bis 6, **dadurch gekennzeichnet, daß** Glukose und Hexokinase in einem Reagenzansatz vorgesehen werden.

## Claims

1. A liquid reagent for the detection of creatine kinase activity, comprising at least the following substances: G6PDH, hexokinase, creatine phosphate, glucose, ADP, NAD(P) and thioglycerol, with the substances being split into two separate batches of reagents and the batches of reagents being mixed for detection purposes, **characterised in, that** G6PDH is contained in one batch, and thioglycerol together with a least one further substance is contained in the other batch.

2. The reagent according to claim 1, **characterised in, that** one of the batches of reagents is adjusted to a weakly acid pH-value between 5.5 and 7.0 and the other batch is adjusted to a weakly basic pH-value between 8.0 to 10.0.

3. The reagent according to claim 2, **characterised in, that** the quantitative ratios and the pH-values of the batches of reagents are selected in such manner that following their mixing, the liquid reagent assumes a pH-value in the range of 6.0 to 7.0, especially a pH-value about 6.5.

4. The reagent according to claims 2 or 3, **characterised in, that** a zwitterionic buffer is present in the basic batch of reagents with its one pK-value situated at pH-values between 8.0 and 10.0 and its other pK-value situated at pH-values between 5.5 and 7.0.

5. The reagent according to claims 2 to 4, **characterised in, that** G6PDH and ADP are present in the batch of reagents having a basic pH-value.

6. The reagent according to claims 1 to 5, **characterised in, that** NAD(P), glucose and G6PDH are not together present in one batch of reagents.

7. The reagent according to claims 1 to 6, **characterised in, that** glucose and hexokinase are present in one batch of reagents.

## Revendications

1. Réactif liquide destiné à la mise en évidence d'une activité de la créatinase et contenant au moins les substances suivantes : G6PDH, hexokinase, créatine-phosphate, glucose, ADP, NAD(P) et thioglycérol, ces substances étant réparties sur deux préparations réactives séparées que l'on mélange pour procéder à la mise en évidence, **caractérisé en ce que** la G6PDH est contenue dans l'une des préparations et le thioglycérol avec au moins une autre substance dans l'autre préparation.

2. Réactif selon la revendication 1, **caractérisé en ce que** le pH de l'une des préparations réactives est ajusté sur une valeur faiblement acide comprise entre 5,5 et 7,0 et celui de l'autre sur une valeur faiblement basique comprise entre 8,0 et 10,0.

3. Réactif selon la revendication 2, **caractérisé en ce que** les quantités relatives et les pH des préparations réactives sont choisis de façon à obtenir, après leur mélange, un réactif d'essai dont le pH se situe entre 6,0 et 7,0 et est notamment d'env. 6,5.

4. Réactif selon l'une des revendications 2 ou 3, **caractérisé en ce que** la préparation réactive basique contient un tampon ionique ampholyte dont l'une des valeurs pK se situe entre pH 8,0 et 10,0 et l'autre entre pH 5,5 et 7,0.

5. Réactif selon l'une des revendications 2 à 4, **caractérisé en ce que** la G6PDH et l'ADP sont contenus dans la préparation de pH basique.

6. Réactif selon l'une des revendications 1 à 5, **caractérisé en ce que** la NAD(P), le glucose et la G6PDH ne sont pas tous contenus ensemble dans la même préparation.

7. Réactif selon l'une des revendications 1 à 6, **caractérisé en ce que** le glucose et l'hexokinase sont contenus dans la même préparation réactive.
